Europäisches Patentamt

European Patent Office

Office européen des brevets

(11) Publication number: **0 047 442**
**A1**

## (12) EUROPEAN PATENT APPLICATION

(21) Application number: **81106654.7**

(22) Date of filing: **27.08.81**

(51) Int. Cl.³: **A 61 M 5/32**

(30) Priority: **04.09.80 US 183916**

(43) Date of publication of application: **17.03.82**
**Bulletin 82/11**

(84) Designated Contracting States: **BE CH DE FR GB IT LI NL SE**

(71) Applicant: **SCHERING CORPORATION, 2000 Galloping Hill Road, Kenilworth, New Jersey 07033 (US)**

(72) Inventor: **Ambrosio, Thomas J., 15 West Cliff Street, Somerville New Jersey 08876 (US)**
Inventor: **Buchman, Henry J., 6 Delores Drive, Edison New Jersey 08817 (US)**
Inventor: **Rogus, Joseph J., 315 Yorktown Road, Bridgewater New Jersey 07103 (US)**
Inventor: **Sochon, Henry R., 205 Haddonfield Road, Clifton New Jersey 07103 (US)**

(74) Representative: **Antony, Fritz, Dr. et al, P.O. Box 601 Winkelriedstrasse 35, CH-6002 Lucerne (CH)**

(54) **Disposable syringe.**

(57) A disposable syringe has a barrel (2), hub (3) and needle (4) with a plunger rod/protector (12) attached to the outer suface (6) of the barrel near the hub by flexible protrusions (16) from the inner surface of a broader end section (14) of the plunger rod/protector. The flexible protrusions frictionally engage the outer surface (6).

Also the plunger rod/protector itself, with the flexible protrusions from the inner surface of a broader end section thereof for gripping the outer surface of the barrel (when present).

Case 2210
July 22, 1981
JB/vl

# DISPOSABLE SYRINGE

This invention relates to disposable syringes and in particular to syringes having a needle protector capable of functioning as a plunger rod after it has been removed from its position protecting the needle and attached to a stopper disposed in the syringe's barrel.

The present invention provides a prefilled disposable syringe comprising:

(a) a barrel, a hub integral with said barrel and a needle rigidly attached to said hub, said barrel having a hollow bore in fluid communication with said needle,

(b) a stopper disposed in said bore and having first engagement means,

(c) injectable medicament disposed in said bore between said stopper and said needle,

(d) sealing means for said needle, and

(e) a plunger rod/protector adapted to enclose said needle and hub and the outer surface of said barrel adjacent to said hub and comprising a narrow portion having a cavity for receiving said needle, said narrow portion able to fit within said hollow bore,

attachment means for attaching said plunger rod/protector to said outer surface at a first end of said narrow portion and comprising an extending broader portion and flexible means protruding therefrom and capable of gripping said outer surface,

and a second engagement means at a second end of said narrow portion adapted to engage said first engagement means,

said plunger rod/protector being adapted to protect said needle during transport and storage and to act as a plunger rod after engagement of said first and second engagement means.

Whereas the extending broader portion may for example take the form of a plurality of fingers around the outer surface of the barrel near the hub, it preferably is of substantially cylindrical shape and thus surrounds and encloses the outer surface and the flexible means protrude therefrom.

Other aspects of the invention comprise the syringe suitable for prefilling and the plunger rod/protector.

For the better understanding of the invention a particularly preferred embodiment thereof will now be described with reference to the accompanying drawings, wherein:

Figure 1 is a longitudinal cross-sectional view of a prefilled disposable syringe in accordance with the invention with the plunger rod/protector and needle sheath shown separated;

Figure 2 is a longitudinal cross-sectional view of the syringe of Figure 1 with the plunger rod/protector and needle sheath attached;

Figure 3 is a partial longitudinal cross-sectional view taken in the longitudinal direction of a plunger rod/protector in accordance with the invention; and

Figure 4 is a view of the plunger rod/protector of Figure 3 taken along line 4-4.

The syringe shown in the drawings has a barrel 2, preferably of glass, with an integral hub 3 and needle 4 rigidly affixed to hub 3. A plunger rod/protector 12 has a narrow portion 9 with a cavity 10 to receive the needle 4. An elastomeric needle-sheath 17 fits freely inside cavity 10 and protects needle 4.

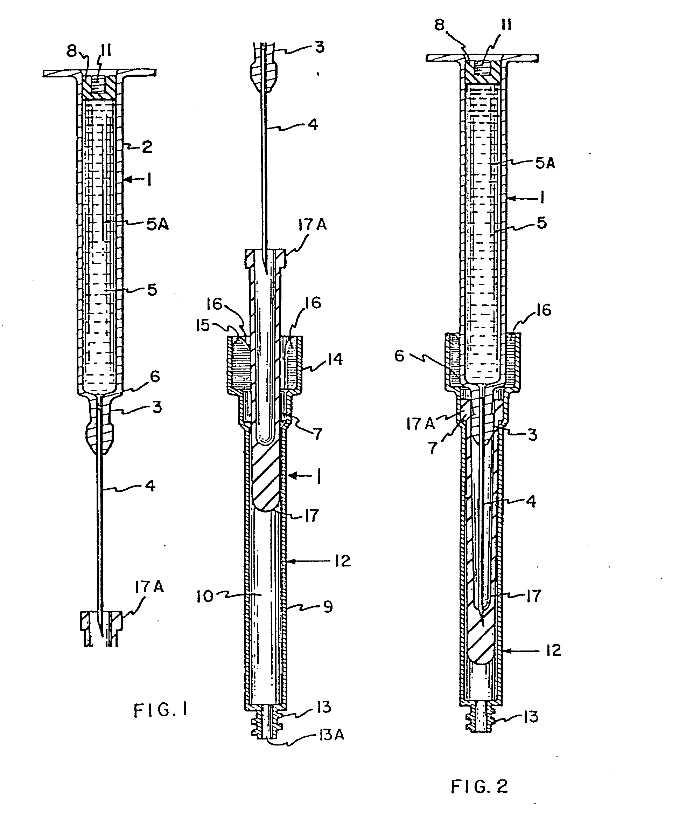

Owing to the length of the arrangement 1 shown in Fig. 1, it has been necessary to divide Fig. 1 into two parts, one part including barrel 2 with integral hub 3 and needle 4, and the other including plunger rod/protector 12 and sheath 17. For the convenience of the reader, both parts of Fig. 1 include the full length of the needle 4.

The barrel 2 has a hollow bore 5 which at one end is in fluid communication with needle 4 and at the other end is closed by a stopper 8. A medicament 5A can be disposed in the hollow bore 5 between the needle 4 and the stopper 8. The stopper 8 is equipped with a first engagement means 11, preferably an inner thread, capable of engaging with a corresponding engagement means 13, preferably an external

- 4 -

thread, on that end of plunger rod/protector 12 remote from needle 4 in the arrangement shown in Fig. 1. The narrow portion 9 is about as long as hollow bore 5, within which it makes a snug sliding fit.

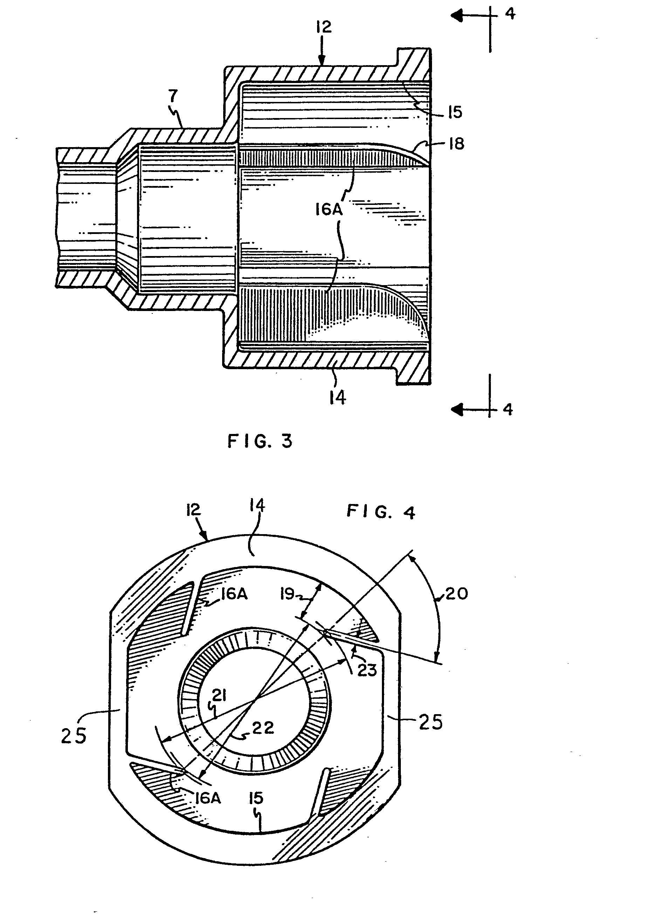

From the end of plunger rod/protector 12 remote from second engagement means 13 there extends a broader portion 14 with an inner surface 15 and flexible protrusions 16, preferably in the form of flexible fins 16A as more clearly shown in Figs. 3 and 4, depending therefrom. The fins 16 allow the sheath 17 to slide comfortably within them but, when the arrangement shown in Fig. 1 is closed up to provide the assembly shown in Fig. 2, they grip barrel 5 at the lower end of its outer surface 6 near hub 3 and thus attach the plunger rod/protector 12 to the barrel 5. In the assembly of Fig. 2, plunger rod/protector 12 encloses needle 4 and hub 3 for protection during shipment and storage. In this position, the sharp end of needle 4 has penetrated sheath 17 and a sterile and hermetic seal is thereby formed. Sheath 17 is held in place by a friction grip at its open end around hub 3. Since the needle sheath 17 normally has a shoulder 17A located at its open end and it is not desired that there should be any grip between the internal surface of plunger rod/protector 12 and needle sheath 17, that part of plunger rod/protector 12 between the narrow portion 9 and the broader portion 14 preferably has a bulge or a zone of intermediate width 7 to accommodate the shoulder 17A of the sheath 17 without gripping it.

The broader portion 14 of plunger rod/protector 12 may be bevelled to provide two diammetrically opposite faces 25, on which information (e.g. batch no.) can be printed and which also serve as a finger grip or to prevent the assembly from rolling when it is laid down. Otherwise, the broader portion 14 of plunger rod/protector 12 is substantially circular, but of course other shapes can be used if desired.

In Figures 3 and 4 the flexible fins 16A are disposed in substantially axial symmetry around the needle (not shown). Fins 16A have rounded corners 17 on their extremities near the open end of the broader portion 14 of plunger rod/protector 12, so that the open arrangement of Fig. 1 can more readily be closed into the assembly of Fig. 2.

Whereas a single fin 16A may provide a sufficient grip between plunger rod/protector 12 and barrel 2, it is preferred to use a plurality of fins more or less symmetrically arranged around the axis of plunger rod/protector 12. So at least three or four fins 16A should preferably be used, with four being most preferred. Angle 20 (Fig. 4), the outer angle between each fin 16A and a radius drawn from the axis of plunger rod/protector 12 through the tip of each fin 16A, may be between $0^\circ$ and $90^\circ$, preferably about $60^\circ$. The plunger rod/protector 12 and its fins 16A should be designed to accommodate syringes of a standard size, for example to accommodate a cylindrical barrel 2 having an outer

- 6 -

diameter 21 (shown by phantom line in Fig. 4) of about
0.425" (10.8 mm.), so that diameter 22 of a circle through
the innermost tip of the fins 16A should be about 0.38" to
0.400" (9.65 to 10.16 mm.).

So that the fins 16A can flex slightly and grip barrel 2
with sufficient resilience, they should have a depth 19
perpendicular to the inner surface 15 of about 0.14" to
0.17" (3.56 to 4.32 mm.), preferably about 0.15" to 0.16"
(3.81 to 4.06 mm.). Especially when the plunger rod/protector is made of plastic, the fins preferably have a thickness 23 of about 0.018" to 0.027" (0.46 to 0.69 mm.), in
particular about 0.022" to 0.025" (0.56 to 0.64 mm.). It
is especially preferred that thickness 23 of fins 16 increase gradually from about 0.02" (0.51 mm.) at rounded corner 18 to about 0.025" (0.64 mm.) at the end of fin 16 adjacent to cavity 7. Surprisingly, fins made to such dimensions are easy to make and hold the barrel 2 snugly and firm-
ly, but are easy to disengage from the barrel 2 when needed,
even after long term storage.

The length of fins 16A, measured in the direction parallel to the longitudinal axis of the plunger rod/protector 12,
should be from 0.3" to 0.5" (7.62 to 12.7 mm.), preferably
about 0.375" (9.53 mm.).

Plunger rod/protector 12 is preferably made of a moulded
plastic material such as polypropylene, polyethylene, polyamide or polyacrylonitrile, or appropriate co-polymers thereof. The mould used to cast the plunger rod/protector 12 and to shape cavity 10 therein preferably has a central rod that is affixed at both ends during casting to prevent it from flexing, so that the cavity cannot become eccentric. This method of casting produces an inner bore 13A in second engagement means 13.

To use the prefilled disposable syringe illustrated in Fig. 2, the operator removes plunger rod/protector 12 from its position covering the needle and engages engagement means 11 and 13. Next he removes sheath 17 and, using plunger rod/protector 12 as a plunger rod for stopper 8, administers the injection in the usual manner.

The present invention has a number of advantages over previously known plunger rod/protectors that attach to the hub by a force fit or an interference fit over a sheath. First, since the plunger rod/protector grips the barrel rather than the hub (even though in most known assemblies the grip on the hub would be an indirect grip through a sheath), it is now much less likely that breakage will occur at the hub, which until now has been the most usual point for breakage to occur. Secondly, since the plunger rod/protector of this invention grips neither the hub nor the needle sheath, the needle is far less likely to be accidentally bent during shipment and handling. Thirdly, since the plunger rod/protector does not grip the needle sheath itself, removal of the plunger rod/protector will leave the sheath in place over the needle so that the needle remains sterile and umcompromised, even if the injection is not immediately administered. Fourthly, the plunger rod/protector, owing to the flexibility of the fins 16A, grips the barrel well despite normal dimensional variations in the size of the barrel.

Previously known assemblies using plunger rod/protectors and barrels, wherein the plunger rod/protector is attached at the hub by a force fit over the sheath, have proved unsatisfactory in a small percentage of cases, owing to variations in size especially in the glass hub. It is difficult to cast glass to close tolerance, and any loose-fitting plunger rod/protectors tend to slip off during shipment and handling. The flexible fins of the present invention can firmly attach themselves directly to the glass surface, despite normal variations in the outer diameter of the glass barrel.

The present invention achieves these advantages without using a special adaptor on the barrel and without causing difficulty in removing the plunger rod/protector from its position covering the needle.

The syringe of the present invention can be filled through the open end of barrel 2 with the sheath 17 in place sealing needle 4; the stopper 8 is then inserted under vacuum. The plunger rod/protector 12 can be placed on the sheath 17 after the filing operation but preferably protects the sheath 17 and needle 4 already during filling.

The invention therefore provides a disposable syringe suitable for prefilling and comprising:

(a) a barrel, a hub integral with said barrel and a needle rigidly attached to said hub, said barrel having a hollow bore in fluid communication with said needle, and

(b) a plunger rod/protector adapted to enclose said needle and hub and the outer surface of said barrel adjacent to said hub and comprising a narrow portion having a cavity for receiving said needle, said narrow portion able to fit within said hollow bore, attachment means for attaching said plunger rod/protector to said outer surface at a first end of said narrow portion and comprising an extending broader portion and flexible means protruding therefrom and capable of gripping said outer surface, and an engagement means at a second end of said narrow portion.

Furthermore the invention provides a plunger rod/protector suitable for use in a disposable syringe as hereinbefore described, comprising a narrow portion having a cavity, attachment means at a first end of said narrow portion comprising an extending broader portion and flexible gripping means protruding therefrom, and an engagement means at a second end of said narrow portion.

CLAIMS

1.    A prefilled disposable syringe comprising:

(a) a barrel, a hub integral with said barrel and a needle rigidly attached to said hub, said barrel having a hollow bore in fluid communication with said needle,

(b) a stopper disposed in said bore and having first engagement means,

(c) injectable medicament disposed in said bore between said stopper and said needle,

(d) sealing means for said needle, and

(e) a plunger rod/protector adapted to enclose said needle and hub and the outer surface of said barrel adjacent to said hub and comprising a narrow portion having a cavity for receiving said needle, said narrow portion able to fit within said hollow bore, attachment means for attaching said plunger rod/protector to said outer surface at a first end of said narrow portion and comprising an extending broader portion and flexible means protruding therefrom and capable of gripping said outer surface, and a second engagement means at a second end of said narrow portion adapted to engage said first engagement means, said plunger rod/protector being adapted to protect said needle during transport and storage and to act as a plunger rod after engagement of said first and second engagement means.

2.    A syringe as claimed in claim 1 wherein said barrel
is of glass and said plunger rod/protector is preferably
constructed of a polymeric material selected from polypropylene, polyethylene, polyamide, polyacrylonitrile,
and appropriate copolymers thereof.

3.    A syringe as claimed in claim 1 or 2 wherein said
extending broader portion surrounds and encloses said
outer surface, and said flexible means, which preferably
comprises a plurality of flexible fins disposed with
substantially axial symmetry around said needle, protrudes
therefrom.

4.    A syringe as claimed in any of claims 1 to 3 wherein
said sealing means (d) comprises an elastomeric needle
sheath that is adapted to cover and seal said needle and
to attach to said hub by a frictional fit at its open end
and preferably has a shoulder at its open end, and wherein
said plunger rod/protector covers said sheath without
engagement therewith and has a bulge between said narrow
portion and said extending broader portion wide enough to
accommodate said shoulder without gripping it.

5.    A syringe as claimed in any of claims 1 to 4 wherein said extending broader portion has an inner surface
from which there protrude three or four fins disposed with substantially axial symmetry to said needle and inclined to the axis of said needle and to said inner surface and preferably having a depth perpendicular to said inner surface of from 3.56 to 4.32 mm. and thickness of from 0.46 to 0.69 mm.

6.    A syringe as claimed in claim 5 wherein said inner surface is approximately circular and said fins protrude therefrom such that the outer angle between each of said fins and a radius of the circle drawn through the tip of each fin is about 60°.

7.    A disposable syringe suitable for prefilling and comprising:

(a)    a barrel, a hub integral with said barrel and a needle rigidly attached to said hub, said barrel having a hollow bore in fluid communication with said needle, and

(b)    a plunger rod/protector adapted to enclose said needle and hub and the outer surface of said barrel adjacent to said hub and comprising a narrow portion having a cavity for receiving said needle, said narrow portion able to fit within said hollow bore, attachment means for attaching said plunger rod/ protector to said outer surface at a first end of said narrow portion and comprising an extending broader portion and flexible means protruding therefrom and capable of gripping said outer surface, and an engagement means at a second end of said narrow portion.

8.    A plunger rod/protector suitable for use in a disposable syringe as claimed in any of claims 1 to 7 and comprising a narrow portion having a cavity, attachment means at a first end of said narrow portion comprising an extending broader portion and flexible gripping means protruding therefrom, and an engagement means at a second end of said narrow portion.

9.    A plunger rod/protector as claimed in claim 8 wherein said flexible means comprises a plurality of flexible fins, preferably at least four fins, disposed with substantially axial symmetry inside said extending broader portion, said fins preferably having a depth, as measured radially to the axis of said extending broader portion, of from 3.56 to 4.32 mm. and thickness of from 0.46 to 0.69 mm.

10.   A plunger rod/protector as claimed in claim 8 or claim 9 further comprising a bulge between said narrow portion and said attachment means having a size intermediate between the size of said attachment means and the cavity in said narrow portion.

0047442

11. A plunger rod/protector as claimed in any of claims 8 to 10 constructed of material selected from polypropylene, polyethylene, polyamide, polyacrylonitrile, and appropriate copolymers thereof.

12. A plunger rod/protector as claimed in claim 11 wherein said extending broader portion has an approximately circular inner surface and wherein said fins protrude therefrom such that an outer angle included between each of said fins and a radius of the circle drawn through the tip of each fin is about $60^{\circ}$.

1/2

FIG. 1

FIG. 2

FIG. 3

FIG. 4

**European Patent Office**

Application number

**EP 81 10 6654**

## EUROPEAN SEARCH REPORT

| | DOCUMENTS CONSIDERED TO BE RELEVANT | | |
|---|---|---|---|
| Category | Citation of document with indication, where appropriate, of relevant passages | Relevant to claim | |
| | US - A - 3 545 607 (KELLER)<br>* Figures 1-3; figures 5-9; column 2, lines 1-15; column 2, lines 66-68; column 3, lines 53-54; column 4, line 19 – column 5, line 3 * | 1,2,4,<br>11 | |
| | -- | | |
| | FR - A - 1 407 737 (NOGIER)<br>* Figures 1,4; page 1, left-hand column, lines 15-25, paragraph 3 * | 2,11 | |

**CLASSIFICATION OF THE APPLICATION (Int. Cl.³)**

A 61 M 5/32

**TECHNICAL FIELDS SEARCHED (Int. Cl.³)**

A 61 M

**CATEGORY OF CITED DOCUMENTS**

X: particularly relevant

A: technological background

O: non-written disclosure

P: intermediate document

T: theory or principle underlying the invention

E: conflicting application

D: document cited in the application

L: citation for other reasons

&: member of the same patent family, corresponding document

The present search report has been drawn up for all claims

| Place of search | Date of completion of the search | Examiner |
|---|---|---|
| The Hague | 01-12-1981 | DURAND-SMET |

EPO Form 1503.1  06.78